# EUROPEAN PATENT APPLICATION

(11) **EP 4 265 632 A1**
(43) Date of publication of application: **25.10.2023**
(21) Application number: 21922304.7
(22) Date of filing: 01.09.2021
(51) Int. Cl.: C07K 14/03, C12N 15/38

(54) **SELF-ASSEMBLED NANOPARTICLE CONTAINING GHGL PROTEIN OF EB VIRUS, AND PREPARATION METHOD THEREFOR AND USE THEREOF**

(30) Priority: 28.01.2021 CN 202110117653
(71) Applicant: Sun Yat-Sen University, Guangzhou, Guangdong 510275 (CN); Sun Yat-Sen University Cancer Center (SYSUCC), Guangzhou Guangdong 510060 (CN)
(72) Inventor: ZENG, Musheng, Guangzhou, Guangdong 510060 (CN); SUN, Cong, Guangzhou, Guangdong 510060 (CN); FENG, Guokai, Guangzhou, Guangdong 510060 (CN); KANG, Yinfeng, Guangzhou, Guangdong 510060 (CN)
(74) Representative: Gassner, Birgitta
(86) International application number: PCT/CN2021/115968
(87) International publication number: WO 2022/160709

(57) **Abstract**

A self-assembled nanoparticle containing a gHgL protein of an EB virus, a preparation method and use thereof. The self-assembled nanoparticle comprises a first polypeptide and a second polypeptide, wherein the first polypeptide comprises a gHgL protein and a first vector subunit, and the second polypeptide comprises a second vector subunit; the first vector subunit is I53-50A1, and the second vector subunit is I53-50B.4PT1; and the gHgL protein is linked to the first vector subunit through a linker. The gHgL protein of the EB virus is displayed on a surface of the self-assembled nanoparticle for the first time. The self-assembled nanoparticle has a larger particle size than the antigen (gHgL), a better antigen residence volume, and a thermal stability comparable to the antigen (gHgL). Moreover, since a larger number of gHgLs are displayed, the self-assembled nanoparticle can strongly stimulate more B cells and induce higher antibody titer. The self-assembled nanoparticle can be used for preventing EB virus infection and treating diseases caused by EB virus infection.

## Description

### TECHNICAL FIELD

This present disclosure belongs to the field of biotechnology and particularly relates to a self-assembled nanoparticle containing a gHgL protein of an EB virus, a preparation method and the use thereof.

### BACKGROUND

EB virus (Epstein-Barr virus, EBV) belongs to γ herpes viruses, which is a double-stranded DNA virus with an envelope. EB virus mainly infects tissues derived from ectoderm, such as skins, membranes and nerves. The EB virus infection is very common in human populations, with a prevalence rate as high as 95%, and it very easily incubates in the body for a lifelong time. In addition to infectious mononucleosis, EB virus may also cause post-transplant lymphoproliferative diseases and some malignant tumors of B cells and epithelial cells. Lymphocytic proliferative diseases include Burkitt lymphoma, diffuse large B-cell lymphoma, NK/T-cell lymphoma, and the like; and malignant tumors include nasopharyngeal carcinoma, gastric cancer, and the like.

A fusion protein complex of EBV comprises both gB and gHgL, which are conserved in the herpesvirus family, and gp350 and gp42, which are specific to EBV It has been found by research that gHgL, as an important receptor-binding protein in the process of membrane fusion, can not only play an independent role in mediating epithelial cell infection, but can also form a common complex with gp42 to participate in B cell infection. In addition, gHgL neutralizing antibodies have very strong EB virus neutralizing effects in both epithelial cell infection and B cell infection, which proves that gHgL is an ideal immunogen for EBV vaccines.

### SUMMARY

In order to overcome the shortcomings of the prior art, a first aspect of the present disclosure aims to provide a self-assembled nanoparticle containing a gHgL protein.

A second aspect of the present disclosure aims to provide a method for preparing the self-assembled nanoparticle of the first aspect.

A third aspect of the present disclosure aims to provide use of the self-assembled nanoparticle of the first aspect in the preparation of a drug for preventing EB virus infection.

A fourth aspect of the present disclosure aims to provide a vaccine comprising the self-assembled nanoparticle of the first aspect as mentioned above.

A fifth aspect of the present disclosure aims to provide use of the self-assembled nanoparticle of the first aspect in the preparation of a drug for treating diseases caused by EB virus infection.

In order to achieve the above objects, the technical solutions used in the present disclosure are as follows.

In a first aspect of the present disclosure, there is provided a self-assembled nanoparticle containing a gHgL protein, which comprises a first polypeptide and a second polypeptide, wherein the first polypeptide comprises a gHgL protein and a first vector subunit, and the second polypeptide comprises a second vector subunit; the first vector subunit is I53-50A1 and the second vector subunit is I53-50B.4PT1; and the gHgL protein is linked to the first vector subunit through a linker, so that the gHgL protein is displayed outside the assembled nanoparticle, and an immune response of a body is better stimulated.

Preferably, the linker comprises a flexible sequence and a rigid connector, and the linker is used for linking the gHgL protein to the vector protein, without affecting the immunogenicity of the gHgL protein and the correct folding of the protein. The vector protein is composed of a first vector subunit and a second vector subunit.

Preferably, the flexible sequence is a polypeptide comprising 5 to 9 amino acids. Furthermore, the flexible sequence is a polypeptide of any one of SEQ ID NO: 12 to SEQ ID NO: 16; moreover, the flexible sequence is a polypeptide as shown in SEQ ID NO: 15.

Preferably, an amino acid sequence of the rigid connector is EKAAKAEEAA (SEQ ID NO: 31).

Preferably, the first vector subunit and the second vector subunit are self-assembled to form a nanostructure by a non-covalent interaction, the first vector subunit is coated on a surface of the second vector subunit, and the gHgL protein is displayed on a surface of the nanostructure.

Preferably, the gHgL protein comprises a gH protein (SEQ ID NO: 28) and a gL protein (SEQ ID NO: 29).

Preferably, the gHgL protein further comprises a linking sequence (SEQ ID NO: 30) for linking the gH protein to the gL protein.

Preferably, an amino acid sequence of the I53-50A1 is shown in SEQ ID NO: 26.

Preferably, an amino acid sequence of the I53-50B.4PT1 is shown in SEQ ID NO: 27.

Preferably, the first polypeptide further comprises a stabilizing protein.

Preferably, the stabilizing protein is located between the linker and the gHgL protein.

Preferably, the stabilizing protein is a T4 fibritin (SEQ ID NO: 32) or a GCN4 peptide fragment (SEQ ID NO: 33); furthermore, the stabilizing protein is a T4 fibritin.

Preferably, the first polypeptide is a first polypeptide trimer.

Preferably, the second polypeptide is a second polypeptide pentamer.

Preferably, a copy number of the first polypeptide trimer is 18-22, and a copy number of the second polypeptide pentamer is 10-14; preferably, the copy number of the first polypeptide trimer is 20, and the copy number of the second polypeptide pentamer is 12.

Preferably, the self-assembled nanoparticle containing the gHgL protein has an icosahedral symmetry.

In a second aspect of the present disclosure, there is provided a method for preparing the self-assembled nanoparticle containing the gHgL protein, comprising incubating the first polypeptide with the second polypeptide to obtain the self-assembled nanoparticle containing the gHgL protein. The first polypeptide comprises a gHgL protein and a first vector subunit, and the second polypeptide comprises a second vector subunit; the first vector subunit is I53-50A1, and the second vector subunit is I53-50B.4PT1; and the gHgL protein is linked to the first vector subunit through a linker, so that the gHgL protein is displayed outside the assembled nanoparticle, and an immune response of a body is better stimulated.

Preferably, the amino acid sequence of the I53-50A1 is shown in SEQ ID NO: 26.

Preferably, the amino acid sequence of the I53-50B.4PT1 is shown in SEQ ID NO: 27.

Preferably, a molar ratio of the first polypeptide to the second polypeptide is 1: (3-6), preferably 1: 5.

Preferably, the incubation is carried out in an assembly buffer for 0.5-2 h.

Preferably, the assembly buffer comprises 250 mM NaCl, 50 mM Tris-HCl with pH 8.0, and 5% glycerol (mass fraction).

Preferably, the gHgL protein comprises a gH protein (SEQ ID NO: 28) and a gL protein (SEQ ID NO: 29).

Preferably, the gHgL protein further comprises a linking sequence (SEQ ID NO: 30) for linking the gH protein to the gL protein.

Preferably, the linker comprises a flexible sequence and a rigid connector, and the linker is used for linking the gHgL protein to the vector protein, without affecting the immunogenicity of the gHgL protein and the correct folding of the protein. The vector protein is composed of a first vector subunit and a second vector subunit.

Preferably, the flexible sequence is a polypeptide comprising 5 to 9 amino acids. Furthermore, the flexible sequence is a polypeptide of any one of SEQ ID NO: 12 to SEQ ID NO: 16; moreover, the flexible sequence is a polypeptide shown in SEQ ID NO: 15.

Preferably, the amino acid sequence of the rigid connector is EKAAKAEEAA (SEQ ID NO: 31).

Preferably, the first polypeptide further comprises a stabilizing protein.

Preferably, the stabilizing protein is located between the linker and the gHgL protein.

The stabilizing protein is preferably a T4 fibritin (SEQ ID NO: 32) or a GCN4 peptide fragment (SEQ ID NO: 33); more preferably, a T4 fibritin.

Preferably, the first polypeptide and the second polypeptide further comprise a purification tag.

The purification tag is preferably at least one selected from the group consisting of histidine tag (His-tag), streptavidin tag (Strep-tag) and maltose binding protein (MBP); more preferably, the purification tag is a histidine tag (His-tag); and most preferably, the purification tag is a histidine tag with an amino acid sequence as shown in SEQ ID NO: 34 or SEQ ID NO: 35.

Preferably, the purification tag of the first polypeptide is located between the stabilizing protein and the linker.

Preferably, the first polypeptide further comprises a linking sequence.

Preferably, the linking sequence is located between the stabilizing protein and the purification tag.

Preferably, the linking sequence is shown in SEQ ID NO: 37.

Preferably, the purification tag of the second polypeptide is located at an end of the second vector subunit.

The first polypeptide further comprises a signal peptide, so that a target protein can be secreted into a supernatant after expression.

The signal peptide is a CD5 signal peptide as shown in SEQ ID NO: 36.

The first polypeptide is preferably obtained by the following steps: introducing a nucleic acid expressing the first polypeptide into a first host cell; and incubating the first host cell to express the first polypeptide.

The first host cell is preferably a eukaryotic cell; more preferably, the first host cell is at least one selected from the group consisting of human embryonic kidney 293 cell (*HEK293F*), a *Madin-Daby canine kidney cell* (*MDCK*), *Chlorocebus sabaeus kidney cell* (*VERO*), *SF9 (Spodoptera frugiperda 9)* cell, *HighFive* cell, a CHO (*Chinese Hamster Ovary*) cell, and yeast cell; more preferably, the first host cell is a human embryonic kidney 293 cell.

The second polypeptide is preferably obtained by the following steps: introducing a nucleic acid expressing the second polypeptide into a second host cell; and incubating the second host cell to express the second polypeptide.

The second host cell is preferably a prokaryotic cell; more preferably, the second host cell is *Escherichia coli;* and most preferably, the second host cell is Rosetta (DE3).

In a third aspect of the present disclosure, there is provided use of the self-assembled nanoparticle of the first aspect in the preparation of a drug for preventing EB virus infection.

In a fourth aspect of the present disclosure, there is provided a vaccine comprising the self-assembled nanoparticle of the first aspect.

A vaccine comprising the above-mentioned self-assembled nanoparticle containing the gHgL protein is provided.

The vaccine further comprises an adjuvant.
the adjuvant is at least one selected from the group consisting of an aluminum adjuvant, an oil emulsion adjuvant such as oil-in-water emulsion, water-in-oil emulsion and bidirectional emulsion, a microorganism-originated adjuvant such as peptidoglycan (PG), lipopolysccharide (LPS) of Gram-negative bacterial outer membrane, mycobacteria and components thereof, GpG oligonucleotide (GpG ODN) and cholera toxin (CT), a microsomal antigen delivery system such as liposome, polymeric microsphere, inert nanosphere, nano aluminum adjuvant, immunostimulating complex (IS-COM), cytokine, a polysaccharide such as inulin (MPI), and a natural source such as propolis and sapoin. More preferably, the adjuvant is a MF59 adjuvant.

In a fifth aspect of the present disclosure, there is provided use of the self-assembled nanoparticle of the first aspect in the preparation of a drug for treating diseases caused by EB virus infection.

The disease is preferably at least one selected from the group consisting of infectious disease, malignant tumor, chronic disease, and autoimmune disease. More preferably, the disease is at least one selected from the group consisting of mononucleosis, nasopharyngeal carcinoma, gastric cancer, epithelial tumors, Burkitt lymphoma, Hodgkin lymphoma, chronic fatigue syndrome, multiple sclerosis, and ankylosing myelitis.

The drug further comprises a pharmaceutically acceptable vector.

The present disclosure has the beneficial effects as follows.

In the self-assembled nanoparticle provided by the present disclosure, the gHgL protein of the EB virus is displayed on the surface of the nanoparticle for the first time. The self-assembled nanoparticle has a larger particle size than the antigen (gHgL), a better antigen residence volume, a thermal stability comparable to the antigen (gHgL). Moreover, since a larger number of gHgLs are displayed, the self-assembled nanoparticle can strongly stimulate more B cells and induce higher antibody titer. The self-assembled nanoparticle can be used for preventing EB virus infection and treating diseases caused by EB virus infection.

Although a heterologous gene is introduced into the self-assembled nanoparticle provided by the present disclosure, since the heterologous gene is derived from a protein of bacteria, which can avoid causing autoimmune diseases, thus having an advantage of high safety without affecting an immune effects.

### BRIEF DESCRIPTION OF DRAWINGS

Fig. 1 is a structural schematic diagram of gHgL-I53-50A1 and gHgL-I53-50 NP, wherein panel (A) is an output structural diagram after Remodel design, in which the distance between gH and I53-50A1 from N-terminal to C-terminal is 31.6 A; panel (B) is a structural fit diagram of the gHgL-I53-50A1 trimer, from which it can be observed that no significant conflict is found in protein chains; and panel (C) is a structural schematic diagram of the gHgL-I53-50 NP nanoparticle, which is the result of the protein structure fitting of the output structure with I53-50ANP (PDB id: 6P6F).
Fig. 2 is a Coomassie brilliant blue staining graph of SDS-PAGE electrophoresis of the self-assembled nanoparticle.
Fig. 3 is a molecular sieve chromatogram of gHgL, a gHgL-I53-50A1 subunit and a gHgL-I53-50 NP self-assembled nanoparticle.
Fig. 4 is a graph showing the dynamic light scattering results of the gHgL, gHgL-I53-50A1 subunit and gHgL-I53-50 NP self-assembled nanoparticle.
Fig. 5 is a negative staining electron micrograph of the gHgL-I53-50 NP self-assembled nanoparticle, wherein panel A is a negative staining electron micrograph of the gHgL-I53-50 NP self-assembled nanoparticle at 200 nm resolution; and panel B is a negative staining electron micrograph of the gHgL-I53-50 NP self-assembled nanoparticle at 100 nm resolution.
Fig. 6 is a diagram showing the differential fluorescence scanning results of the gHgL, gHgL-I53-50A1 subunit and gHgL-I53-50 NP self-assembled nanoparticle.
Fig. 7 is bio-layer interferometry graphs of the gHgL, gHgL-I53-50A1 subunit and gHgL-I53-50 NP self-assembled nanoparticle against the neutralizing antibody AMMO1, wherein panel A is a bio-layer interferometry graph of gHgL against the neutralizing antibody AMMO1; panel B is a bio-layer interferometry graph of the gHgL-I53-50A1 subunit against the neutralizing antibody AMMO1; and panel C is a bio-layer interferometry graph of the gHgL-I53-50 NP self-assembled nanoparticle against the neutralizing antibody AMMO1.
Fig. 8 is a graph showing the total antibody titer of serum gHgL in mice after immunization, wherein panel A is a graph showing the total antibody titer of serum gHgL at week 2 after immunization, and panel B is a graph showing the total antibody titer of serum gHgL at week 5 after immunization, with ** in the drawing representing P < 0.005.

### DETAILED DESCRIPTION

The content of the present disclosure will be further illustrated in detail in conjunction with specific examples and drawings.

It should be understood that these examples are only used to illustrate the present disclosure and are not used to limit the scope of the present disclosure.

In the following examples, the experimental methods in which no specific conditions are specified are usually in accordance with conventional conditions. The common chemical reagents used in the examples are all commercially available products.

The method for preparing the nanoparticle vaccine of the present disclosure includes the following steps.
A. by means of a computer-aided design such as Rosetta, determining the fusion compatibility of gHgL with a trimer stabilizing protein is determined, and an expression sequence is designed according to the results.
B. An eukaryotic expression vectors are transferred into a host first cell for expression by a transient transfection technology to obtain a nanoparticle subunit protein of gHgL-I53-50A1 (a first polypeptide). Meanwhile, the expression plasmid of I53-50B.4PT1 is transformed into a second host cells, and after induction with IPTG, another nanoparticle subunit protein of I53-50B.4PT1 (a second polypeptide) is expressed and obtained. The both two proteins are subjected to affinity chromatography and molecular exclusion chromatography for further purification and then to SDS-PAGE gel electrophoresis to identify the purity thereof.
C. gHgL-I53-50A1 and I53-50B.4PT1 subunits are added into an assembling buffer according to a certain proportion, and incubated at a room temperature to obtain an assembled nanoparticle. The assembled nanoparticle is separated by molecular exclusion chromatography, and particle size distribution and stability of the protein is determined by negative staining electron microscopy, dynamic light scattering and differential scanning fluorescence.
D. An antigenicity of the nanoparticle is determined by bio-layer interferometry (BLI).
E. The nanoparticle is evenly mixed with an adjuvant, and a Balb/C mouse is immunized to verify an antibody level against gHgL generated in the mouse.

The nanoparticle vaccine of the present application is further described in detail hereinafter.

### Example 1 Linker design

By means of a computer software-aided design such as Rosetta, domain insertion design was carried out by means of rosetta remodel software, and a trimer stabilizing protein was structurally linked to gHgL antigen (SEQ ID NO: 1), so as to judge whether it was necessary to insert a linker. Finally, structure visualization was carried out by means of PyMol for visual judgment, whereby such linkers that were composed of a flexible sequence and a rigid connector were eventually selected. The various linkers were only different in the flexible sequence. The amino acid sequences and nucleotide sequences of the flexible sequences of the various linkers were as shown in Table 1, where the amino acid sequence of the rigid connector was shown in SEQ ID NO: 31, and the nucleotide sequence thereof was shown in SEQ ID NO: 17.

Softwares used in the design:
https://www.rosettacommons.org/docs/latest/application_documentation/design/rosettare model; and
Pymol open-source: https://github.com/schrodinger/pymol-open-source.

### Example 2 Recombinant vector construction and protein expression

### 1. Experimental materials

(1) Expression vector: eukaryotic expression vector: pcDNA3.1(+) (ThermoFisher), prokaryotic expression vector: pET28a(+) (ThermoFisher), and *Escherichia coli* competent cells DH5α (Tiangen).
(2) Expression system: eukaryotic expression system cells HEK93F (ATCC) and transformed *Escherichia coli* cell Rosetta (DE3) (Tiangen).
(3) Reagents and consumables: PCR enzyme (GeneStar), recombinant enzyme (Vazyme), restriction endonuclease (NEB), gel recovery agent (Genestar), plasmid midiprep kit (MN), cell transfection reagent PEI (Polyscience), 293F medium (Union), TB culture medium (Xiangbo Bio), purified agarose beads of histidine tag protein (Roche), and other conventional reagents and materials purchased commercially.
(4) Genes: gH and gL genes of EB virus (M81 strain) and I53-50A1/I53-50B particle subunit genes optimized based on bacterial protein, which were all optimized and synthesized through the OptimumGene^{™} codon platform of Nanjing GenScript Biological Co., Ltd..

### 2. Linker screening

We had tried various linkers, which were composed of a flexible sequence and a rigid connector. The various linkers were only different in the flexible sequence. Similarly, after an expression vector was constructed and transfected for expression, the protein concentration was determined after purification and concentration. The specific steps were as follows: (1) a gH gene of EB virus (SEQ ID NO: 2), a linking sequence (SEQ ID NO: 3), a gL gene (SEQ ID NO: 4), a T4 fibritin (SEQ ID NO: 5), a I53-50A1 (SEQ ID NO: 6) and a linker (the amino acid sequences and nucleotide sequences of the flexible sequences of various linkers were as shown in Table 1, and the nucleotide sequence of the rigid connector was shown in SEQ ID NO: 17) were inserted into the vector PCDN3.1(+) by PCR amplification and enzyme digestion recombination, so as to obtain the target gene gHgL-I53-50A1 expressed by the expression vector. Wherein, the front end of the vector pcDNA3.1(+) was provided with a CD5 signal peptide (SEQ ID NO: 18) for secreting the expressed polypeptide outside cells, there was an eight-histidine His-tag (SEQ ID NO: 19) between T4 fibritin and the linker for convenient purification, and the front end of the His-tag was connected to a linking sequence (SEQ ID NO: 20). (2) The recombinant vector gene in pcDNA3.1 was transformed into DH5α competent bacteria, and positive clones were screened by ampicillin resistance. Then, the positive clones were picked into a TB culture medium containing 0.1% ampicillin (0.1 mg/mL) for amplification, and then extracted by using the midiprep kit. The specific method could be referred to the instruction of product. (3) 293F cells were subjected to suspension culture and amplification in in a 293F medium (Union), and were ready for transient transfection after being amplified to a certain quantity. The cells were diluted to 1 L with a density of 1^{∗}10⁶/mL, and then, a transfection system of 1 mg of pcDNA3.1-target protein vector 5 mg PEI was prepared with a fresh medium, added into the diluted 293F cells after standing for 30 min, and cultured at 37°C, 30% humidity, 5% CO₂ concentration for 7 days under shaking at 120 rpm. The cell precipitate was removed by centrifugation. The supernatant was filtered with a 0.22 µm filter membrane, and then purified by protein affinity chromatography and molecular sieve to obtain a high-purity target protein gHgL-I53-50A1 subunit.

The results were as shown in Table 1. When the flexible sequence of the linker was GGSGGSGS (SEQ ID NO: 15), the yield of the gHgL-I53-50A1 subunit was the highest.

**Table 1 Yield of proteins from vectors with linkers having various flexible sequences**

| Flexible sequence (nucleic acid sequence) | Flexible sequence (amino acid sequence) | Length of amino acid sequenc e | Yield (mg/L medium) |
|---|---|---|---|
| GGAGGAAGCGGAAGC (SEQ ID NO:7) | GGSGS(SEQ ID NO: 12) | 5 | 0.24 |
| GGAGGAAGCGGAGGCTCT (SEQ ID NO:8) | GGSGGS (SEQ ID NO:13) | 6 | 0.21 |
| GGAGGAAGCGGAGGCTCTGGA (SEQ ID NO:9) | GGSGGSG (SEQ ID NO: 14) | 7 | 0.38 |
| GGAGGAAGCGGAGGCTCTGGAA GC (SEQ ID NO: 10) | GGSGGSGS (SEQ ID NO:15) | 8 | 1.2 |
| GGAGGAAGCGGAGGCTCTGGAG GCTCT (SEQ ID NO:11) | GGSGGSGGS (SEQ ID NO:16) | 9 | 1.0 |

### 3. Steps of preparation of self-assembled nanoparticles

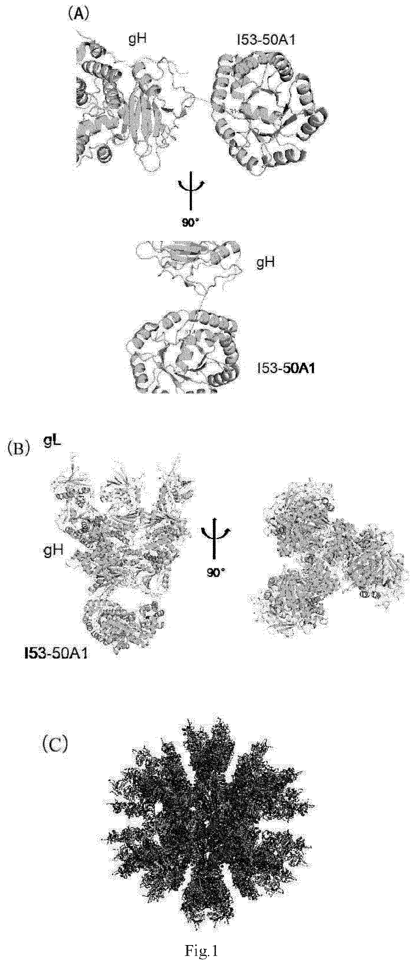

(1) A gH gene of EB virus (SEQ ID NO: 2), a linking sequence (SEQ ID NO: 3), gL gene (SEQ ID NO: 4), T4 fibritin (SEQ ID NO: 5), I53-50A1 (SEQ ID NO: 6) and a linker (the nucleotide sequence of the flexible sequence of the linker was as shown in SEQ ID NO: 10 and the nucleotide sequence of the rigid connector was shown in SEQ ID NO: 17) were inserted into the vector PCDN3.1(+) by PCR expansion and enzyme digestion recombination, so as to obtain the target gene gHgL-I53-50A1 (SEQ ID NO: 21) expressed by the expression vector. The front end of the vector pcDNA3.1(+) was provided with a CD5 signal peptide (SEQ ID NO: 18) for secreting the expressed polypeptide outside cells, there was an eight-histidine His-tag (SEQ ID NO: 19) between T4 fibritin and the linker for convenient purification, and the front end of the His-tag was connected to a linking sequence (SEQ ID NO: 20). In addition, I53-50B.4PT1 (SEQ ID NO: 22) was directly inserted into the pET28a(+) vector during synthesis, and a six-histidine His-tag (SEQ ID NO: 23) was provided at the tail end for convenient purification. After sequencing and comparison, a successfully constructed vector was selected for the next step of experiment.
(2) The recombinant vector gene in pcDNA3.1 was transformed into DH5α competent bacteria, and the positive clones were screened by ampicillin resistance. Then, the positive clones were picked into a TB culture medium containing 0.1% ampicillin (0.1 mg/mL) for amplification, and then extracted by using a midiprep kit. The specific method could be referred to the instruction of product.
(3) The recombinant vector gene in pET28a(+) was transformed into Rosetta (DE3) competent bacteria, and positive clones were screened by kanamycin resistance. Then, the positive clones were picked into a TB culture medium containing 0.1% kanamycin (0.03 g/mL) for amplification, and then further amplified to 1 L in a conical flask, and kanamycin and chloramphenicol were added for screening positive cells. 0.2 mM chemical inducer isopropyl thiogalactoside (IPTG) was added at 18°C to induce expression of the target protein, and after induction for 20 hours, bacterial cells were collected, crushed under a high pressure, and centrifuged to obtain a supernatant. The supernatant was obtained filtered at 0.22 µm filtration, and purified by protein affinity chromatography and molecular sieve to obtain a high-purity target protein I53-50B.4PT1 subunit (SEQ ID NO: 24) with a His-tag.
(4)The 293F cells were subjected to suspension culture and amplification in a 293F medium (Union), and were ready for transient transfection after being amplified to a certain quantity. The cells were diluted to 1 L with a density of 1^{∗}10⁶/mL, and then, a transfection system of 1 mg of pcDNA3.1-target protein vector 5 mg PEI was prepared with a fresh medium, added into the diluted 293F cells after standing for 30 min, and cultured at at 37°C, 80% humidity, 5% CO₂ concentration for 7 days under shaking at 120 rpm. The cell precipitate was removed by centrifugation. The supernatant was filtered with a 0.22 µm filter membrane, and then purified by protein affinity chromatography and molecular sieve to obtain the high-purity target protein gHgL-I53-50A1 subunit (SEQ ID NO: 25) with a His-tag. The structural schematic diagram of gHgL-I53-50A1 is as shown in Fig. 1.
(5) Two histidine-tagged subunits (gHgL-I53-50A1 and I53-50B.4PT1) were added to an assembly buffer (250 mM NaCl, 50 mM Tris-HCl with pH 8.0, and 5% glycerol (mass fraction)) at a molar ratio of 1: 5, and incubated at room temperature for 1 h, and then the assembled nanoparticles (gHgL-I53-50 NP) were separated by means of molecular sieves. The structural schematic diagram of the gHgL-I53-50 NP is as shown in Fig. 1.

### 4·Results

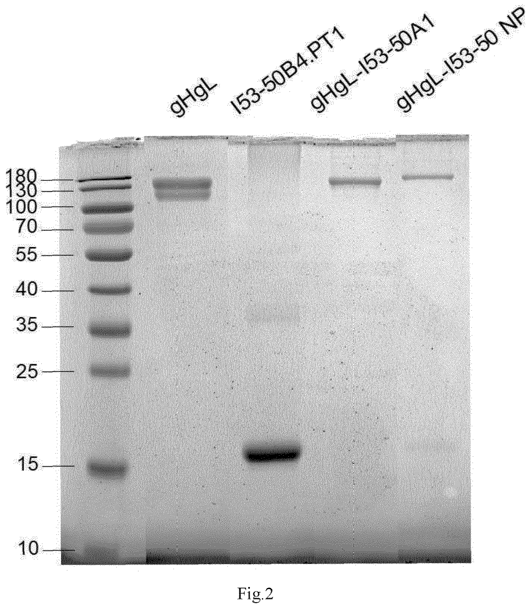

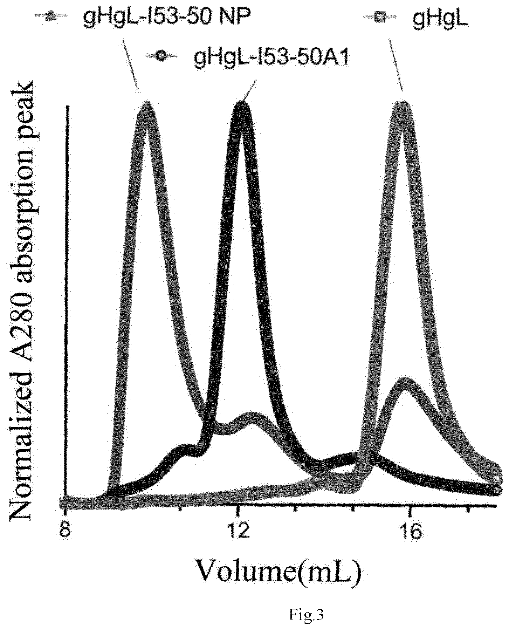

As shown in Fig. 2 and Fig.3, Fig. 2 shows the results of Coomassie brilliant blue staining by SDS-PAGE electrophoresis of nanoparticles: from left to right, gHgL antigen protein (SEQ ID NO: 38, the preparation method of which was the same as the preparation method of the gHgL-I53-50A1 subunit in Point 3, only except that in step (1), T4 fibritin (SEQ ID NO: 5), I53-50A1 (SEQ ID NO: 6), linking sequence (SEQ ID NO: 20) and linker (the nucleotide sequence of the flexible sequence of the linker was shown in SEQ ID NO: 10, and the nucleotide sequence of the rigid connector was shown in SEQ ID NO: 17) were not inserted into the vector pcDNA3.1(+)), an I53-50B.4PT1 subunit, a gHgL-I53-50A1 subunit (the preparation method of which was the same as the preparation method for I53-50B.4PT1 subunit and gHgL-I53-50A1 subunit in Point 3) and the gHgL-I53-50 NP self-assembled nanoparticles obtained in point 3. Fig. 3 is a molecular sieve chromatogram of the nanoparticles, from which it can be seen that the recombinant vector is successfully constructed, and a high-purity nanoparticle protein (gHgL-I53-50 NP) can be obtained. The molecular mass of gHgL-I53-50A1 is larger than that of gHgL.

### Example 3 Detection of structural characteristics and chemical stability of nanoparticles

### 1. Experimental materials

(1) Unchained Uncle high-throughput protein stability analyzer (Unchained Labs).
(2) 120 KV transmission electron microscope (FEI).

### 2. Experimental steps

(1) Detection of particle size distribution of nanoparticles
   The gHgL self-assembled nanoparticle (gHgL-I53-50 NP), gHgL-I53-50A1 subunit and antigen protein (gHgL) in Example 2 were diluted to 0.5 mg/mL. 200 uL of the samples were then added to a special sample loading slot of Uncle, and stood for 5 min, and then the particle size of the nanoparticles was detected by an Uncle instrument from Unchained Company.
(2) Detection of structural characteristics of nanoparticles
   The gHgL self-assembled nanoparticles (gHgL-I53-50 NP) in Example 2 was diluted to a concentration of 0.1 mg/mL. The protein was incubated on a carbon-coated copper grid, then incubated and stained with 2% uranium acetate for 2 minutes, and dried in air. Then, the particle size and morphology of the particle were observed by using 120 KV transmission electron microscope.
(3) Detection of thermal stability of nanoparticles
   The gHgL self-assembled nanoparticles (gHgL-I53-50 NP), gHgL-I53-50A1 subunit and antigen (gHgL) in Example 2 were diluted to a concentration of 0.5 mg/mL. Then the heating scanning was carried out from 25°C to 90°C by using an Uncle instrument from Unchained Company, and the change of the full-wavelength broad-spectrum shift (BCM) was recorded.

### 3. Experimental results

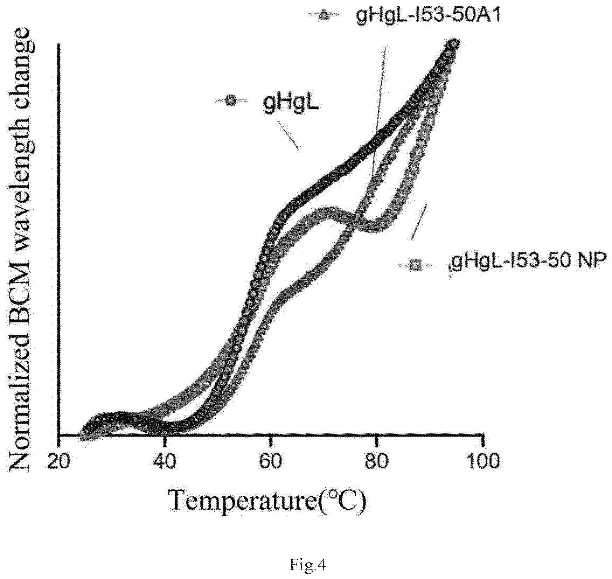

As shown in Fig. 4, the gHgL self-assembled nanoparticles (gHgL-I53-50 NP) has a uniform particle size distribution characteristic, and the particle size thereof is significantly larger than those of the gHgL-I53-50A1 subunit and the antigen (gHgL), indicating that nanoparticles have been assembled successfully.

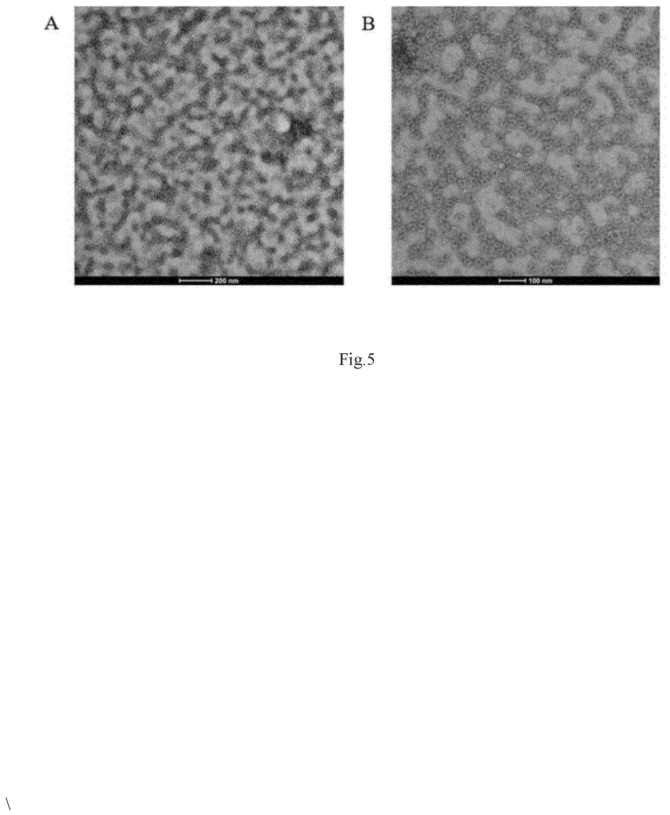

As shown in Fig. 5, it can be seen under negative staining electron microscope that gHgL-I53-50 NP has a relatively good uniformity, and there are obvious external protrusions on the particle surface of gHgL-I53-50 NP, indicating that gHgL is successfully displayed on the surface of the nanoparticle vector.

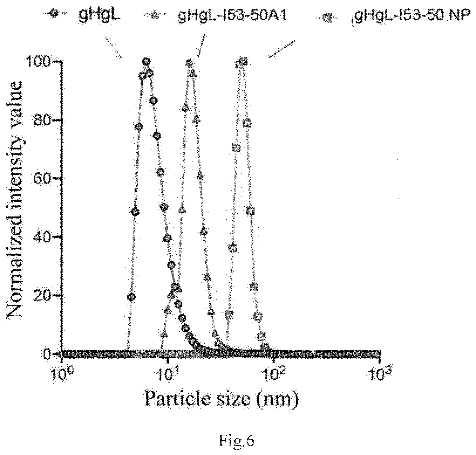

As shown in Fig. 6, Fig. 6 showes the results of the differential fluorescence scanning of gHgL, gHgL-I53-50A1 and gHgL-I53-50 NP. As the temperature rises from 25°C to 95°C, the BCM shifts of the three are similar, confirming that the modification had no significant influence on the stability of the protein gHgL itself. In addition, since gHgL-I53-50 NP has nanoparticle characteristics, the slope of fluorescence change is also smaller than that of gHgL.

### Example 4 Antigenicity of nanoparticles

### 1. Experimental materials

(1) ProteinA sensor (Fortebio), PBS, and Tween 20 (Sigma-Aldrich).
(2) Fortebio Octet 96 instrument.
(3) Pre-wetted plate, 96-well plate, and other commercial and conventional consumables.

### 2. Experimental steps

### (1) Detection of affinity between nanoparticles and neutralizing antibody by BLI

0.5% PBST was prepared for kinetic detection. 150 uL of PBST was added into the pre-wetted plate, and incubated in the proteinA sensor for 10 minutes. The antibody AMMO1 (for the preparation method, see the reference Snijder et al., 2018, Immunity 48, 799-811) was then diluted for coupling. After equilibrium, coupling was started, and the antigens such as nanoparticle proteins (gHgL, gHgL-I53-50A1 and gHgL-153-50 NP in Example 2) were then diluted in a gradient (3.125 nM, 6.25 nM, 12.5 nM, 25 nM, 50 nM, 100 nM, and 200 nM), and bound to the sensor. The binding signal and dissociation signal were recorded, and the sensor was regenerated by suing a glycine solution. The binding signal was fitted by using a binding model of 1: 1 to calculate the dynamic parameters.

### 3. Experimental results

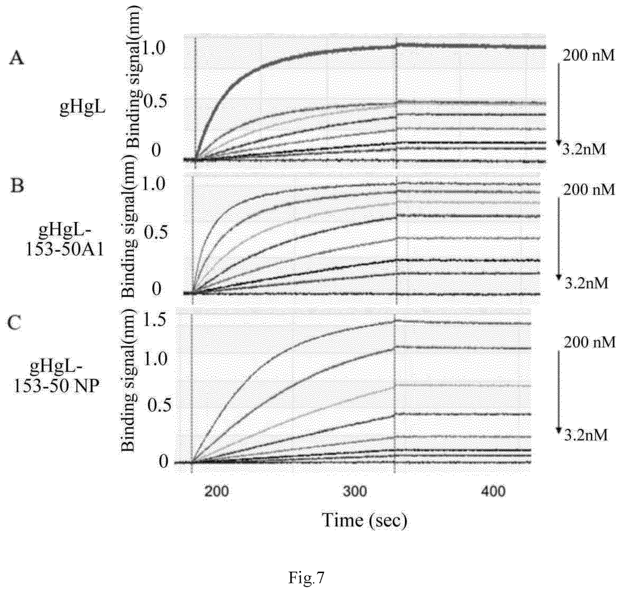

As shown in Fig. 7 and Table 2, the binding capacity of the gHgL nanoparticles (gHgL-I53-50 NP) to the AMMO1 antibody is stronger than that of gHgL, demonstrating that the antigenicity of the gHgL nanoparticles (gHgL-I53-50 NP) is stronger than that of gHgL. This characteristic contributes to residence on BCR (B cell antigen receptor) for a long time and stimulation of antibody production.

**Table 2 Antibody affinity kinetic parameters of gHgL, gHgL-I53-50A1 and gHgL-I53-50 NP**

| | KD (M) |
|---|---|
| gHgL | 4.32E-10 |
| gHgL-I53-50A1 | 2.42E-10 |
| gHgL-I53-50 NP | 3.39E-11 |

### Example 5 Immunogenicity of nanoparticle protein in animals

### 1. Experimental materials

(1) Mice: BalB/C mice, female, 6 weeks to 8 weeks of age (Beijing Charles River Laboratory Animal Technology Co., Ltd.).
(2) Adjuvant: MF59 Adjuvant {0.5% (v/v) Tween 80, 0.5% (v/v) Span 85, 4.3% (v/v) squalene, and 10 nM sodium citrate buffer}.
(3) Other commercial and conventional reagent.

### 2. Experimental steps

(1) 2 ug of empty nanovector (empty-NP, the preparation method was the same as Point 3 in Example 2, only except that gH gene (SEQ ID NO: 2), linking sequence (SEQ ID NO: 3), gL gene (SEQ ID NO: 4), T4 fibritin (SEQ ID NO: 5), linker and linking sequence (SEQ ID NO: 20) were not contained), 2 ug of gHgL protein of EB virus (gHgL prepared in Example 2), and gHgL nanoparticles containing the same molar mass of gHgL (gHgL-I53-50 NP in Example 2) were respectively mixed with the above MF59 adjuvant, that is, the adjuvant were mixed with the antigen at a mass ratio of 1 : 1, and incubated under shaking overnight at 4°C. The mice were immunized by subcutaneous immunization.
(2) The mice were immunized again at week 3 after immunization. The orbital blood was collected from the mice at weeks 2 and 6 after immunization, and separated to collect serum. The total antibody titer of gHgL in the serum of the mice was detected by indirect enzyme-linked immunosorbent assay.

### 3. Experimental results

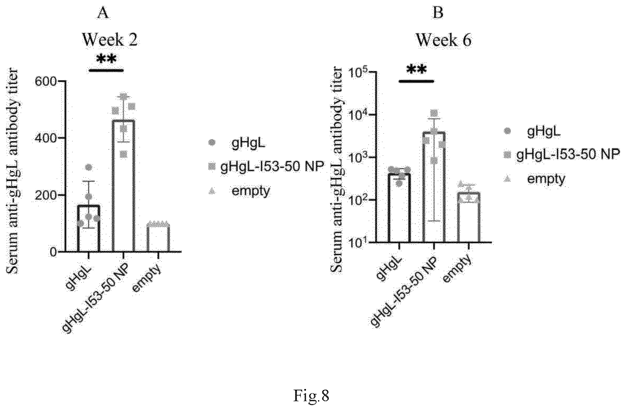

As shown in Fig. 8, during the detection of the serum antibody titers at both weeks 2 and 6, the total serum antibody titer induced by gHgL self-assembled nanoparticles (gHgL-I53-50 NP) is higher than that induced by monomeric gHgL, confirming that the gHgL nanoparticles can induce stronger antibody production.

The above examples are preferred embodiments of the present disclosure, and the embodiments of the present disclosure are not limited by the above examples. Any other changes, modifications, substitutions, combinations and simplifications made without departing from the spirit essence and principle of the present disclosure shall be equivalent substitutions and are all included in the scope of protection of the present disclosure.

## Claims

1. A self-assembled nanoparticle, comprising a first polypeptide and a second polypeptide, wherein the first polypeptide comprises a gHgL protein and a first vector subunit, and the second polypeptide comprises a second vector subunit; the first vector subunit is I53-50A1, and the second vector subunit is I53-50B.4PT1; and the gHgL protein is linked to the first vector subunit through a linker.

2. The self-assembled nanoparticle according to claim 1, wherein,
an amino acid sequence of the I53-50A1 is shown in SEQ ID NO: 26;
an amino acid sequence of the I53-50B.4PT1 is shown in SEQ ID NO: 27; and
preferably, the linker comprises a flexible sequence and a rigid connector,
the flexible sequence is a polypeptide comprising 5 to 9 amino acids and an amino acid sequence of the rigid connector is EKAAKAEEAA.

3. The self-assembled nanoparticle according to claim 2, wherein,
the first polypeptide further comprises a stabilizing protein;
the stabilizing protein is located between the linker and the gHgL protein; and
preferably, the stabilizing protein is a T4 fibritin or a GCN4 peptide fragment.

4. The self-assembled nanoparticle according to claim 3, wherein, the first polypeptide is a first polypeptide trimer and the second polypeptide is a second polypeptide pentamer.

5. The self-assembled nanoparticle according to claim 4, wherein a copy number of the first polypeptide trimer is 18-22, and a copy number of the second polypeptide pentamer is 10-14.

6. The self-assembled nanoparticle according to any one of claims 1-5, wherein,
the gHgL protein comprises a gH protein and a gL protein; and
preferably, the gHgL protein further comprises a linking sequence.

7. A method for preparing the self-assembled nanoparticle according to any one of claims 1-6, comprising incubating the first polypeptide with the second polypeptide,
preferably, a molar ratio of the first polypeptide to the second polypeptide is 1 : (3-6).

8. Use of the self-assembled nanoparticle according to any one of claims 1-6 in the preparation of a drug for preventing EB virus infection.

9. A vaccine, comprising the self-assembled nanoparticle according to any one of claims 1-6; and
the vaccine preferably further comprises an adjuvant.

10. Use of the self-assembled nanoparticle according to any one of claims 1-6 in the preparation of a drug for treating diseases caused by EB virus infection.
